# EUROPEAN PATENT APPLICATION

(11) **EP 4 356 940 A1**
(43) Date of publication of application: **24.04.2024**
(21) Application number: 22824553.6
(22) Date of filing: 09.03.2022
(51) Int. Cl.: A61M 1/36, A61M 60/109, A61M 60/279, A61M 60/37

(54) **BLOOD PURIFICATION DEVICE**

(30) Priority: 14.06.2021 JP 2021098773
(71) Applicant: Nikkiso Co., Ltd., Tokyo 150-6022 (JP)
(72) Inventor: MAEDA, Takuya, Makinohara-shi, Shizuoka 421-0496 (JP); TSUJI, Kazuya, Makinohara-shi, Shizuoka 421-0496 (JP)
(74) Representative: von Hirschhausen, Helge
(86) International application number: PCT/JP2022/010393
(87) International publication number: WO 2022/264553

(57) **Abstract**

Provided is a blood purification apparatus that drains a residual liquid in a circuit. The blood purification apparatus includes: a sealed blood circuit including a blood removal side circuit and a blood return side circuit connected to each other; a drainage circuit that is connected to the blood circuit and drains a residual liquid in the blood circuit; an air introducer that is connected to the blood circuit and introduces air into the blood circuit; and a liquid delivery pump that is provided to at least one of the blood circuit and the drainage circuit and, by rotating, delivers the residual liquid to the drainage circuit from the blood circuit with the introduced air as a medium for pushing out the residual liquid.

## Description

### Technical Field

The present disclosure relates to a blood purification apparatus.

### Background Art

In a case where kidneys that constitute part of the organs of a human do not operate normally (renal failure), functions to produce urine out of excess water in the body, to excrete unwanted waste products in the body, and so forth do not work well. To deal with the renal failure, a blood purification apparatus (dialysis apparatus) is used for conducting a treatment to extracorporeally circulate blood from the patient and to filter out waste products and water in the blood by using a blood purifier (dialysis treatment).

In the dialysis treatment, the blood purification apparatus withdraws blood from the patient and introduces the blood into the blood purifier through a blood circuit while introducing a dialysate from a dialysate supply source (dialysate supplier) into the blood purifier through a dialysate circuit at the same time. Then, the blood purification apparatus purifies the blood by exchanging components such as waste products and electrolytes between the blood and the dialysate through the blood purifier, and returns the purified blood to the body. The blood circuit includes a circuit connected to an arterial side of the patient through the blood purifier (arterial side blood circuit) and a circuit connected to a venous side of the patient through the blood purifier (venous side blood circuit).

### Citation List

### Patent Literature

PTL 1: Japanese Patent No. 4160754

### Summary of Invention

### Technical Problem

After the dialysis treatment, a process of draining the liquid remaining in the blood circuit (residual liquid) (drainage process) is performed. There is a technique of draining the residual liquid in the drainage process by coupling the arterial side blood circuit and the venous side blood circuit so as to form the blood circuit into a closed loop. PTL 1 discloses a technique employing the above-described configuration to drain a residual liquid in a blood circuit.

In the technique disclosed in PTL 1, the blood circuit is closed and a blood pump is actuated to dispose of the residual liquid in the closed loop through a drainage pipe. The technique disclosed in PTL 1 employs a blood circuit formed from a sealed tube through which no air flows to / from the blood circuit (it is apparent that the blood circuit is formed from a sealed tube from the description of paragraph 0027 "The flexible bag 27 is rapidly emptied and collapses").

Moreover, in the technique disclosed in PTL 1, simply the blood pump (i.e., a liquid delivery pump for delivering a residual liquid in a circuit) is actuated to drain the residual liquid in the closed loop (in other words, to push out the liquid only with a pressure generated by driving the pump). Hence, the closed loop contains no medium for pushing out the residual liquid (i.e., air). In the technique disclosed in PTL 1, the pressure inside a compartment 5 is made equal to the atmospheric pressure. However, this is meant to introduce air to a dialyzer including two compartments, and is not meant to introduce air into the blood circuit. In other words, PTL 1 does not clearly describe a configuration for introducing air into the blood circuit in a case of draining the residual liquid in the blood circuit.

With a configuration employing a blood circuit formed from a sealed tube, in a case of only driving a liquid delivery pump in a drainage process, the residual liquid in the circuit is drained without introducing the above-mentioned medium into the blood circuit (in other words, the blood circuit gets close to a vacuum state). Thus, only driving the liquid delivery pump may result in a failure to sufficiently push out the residual liquid. Therefore, in the technique disclosed in PTL 1, the liquid delivery pump needs to be driven at a high output power in order to drain the residual liquid in the closed loop. An object of the embodiments is to provide a blood purification apparatus capable of draining a residual liquid in a blood circuit in a drainage process without driving a liquid delivery pump at a high output power.

### Solution to Problem

A blood purification apparatus according to an embodiment is a blood purification apparatus that drains a residual liquid in a circuit, including: a sealed blood circuit including a blood removal side circuit and a blood return side circuit connected to each other; a drainage circuit that is connected to the blood circuit and drains a residual liquid in the blood circuit; an air introducer that is connected to the blood circuit and introduces air into the blood circuit; and a drainage pump that is provided to at least one of the blood circuit and the drainage circuit and, by rotating, drains the residual liquid to the drainage circuit from the blood circuit with the introduced air as a medium for pushing out the residual liquid.

A method according to another embodiment is a method of draining a residual liquid in a circuit executed by a blood purification apparatus, the blood purification apparatus including: a sealed blood circuit including a blood removal side circuit and a blood return side circuit connected to each other; and a drainage circuit that is connected to the blood circuit and drains a residual liquid in the blood circuit, the method including the steps of: introducing air into the blood circuit; and delivering the residual liquid to the drainage circuit from the blood circuit with the introduced air as a medium for pushing out the residual liquid.

### Advantageous Effects of Invention

In the blood purification apparatus according to the embodiment, the air introduced into the blood circuit plays a role as a medium for pushing out the residual liquid. Thus, the residual liquid in the blood circuit can be drained by driving the liquid delivery pump at a lower output power than that in the technique disclosed in PTL 1.

### Brief Description of Drawings

[Fig. 1] Fig. 1 is a circuit diagram illustrating a configuration of a blood purification apparatus according to a first embodiment.
[Fig. 2] Fig. 2 is a diagram illustrating operations of constituents in a case of performing dialysis treatment according to the first embodiment.
[Fig. 3] Fig. 3 is a diagram illustrating the operations of the constituents in the case of performing a drainage process according to the first embodiment.
[Fig. 4] Fig. 4 is a diagram illustrating the operations of the constituents in the case of performing a drainage process according to the first embodiment.
[Fig. 5] Fig. 5 is a diagram illustrating operations of constituents in a case of performing a drainage process according to a second embodiment.
[Fig. 6] Fig. 6 is a diagram illustrating the operations of the constituents in the case of performing a drainage process according to the second embodiment.
[Fig. 7] Fig. 7 is a circuit diagram illustrating a configuration of a blood purification apparatus according to a third embodiment.
[Fig. 8] Fig. 8 is a flowchart illustrating a process by the blood purification apparatus according to the third embodiment.
[Fig. 9] Fig. 9 is a diagram illustrating operations of constituents in a case of performing a drainage process according to the third embodiment.
[Fig. 10] Fig. 10 is a flowchart illustrating a process by a blood purification apparatus according to a fourth embodiment.
[Fig. 11] Fig. 11 is a diagram illustrating operations of constituents in a case of performing a drainage process according to the fourth embodiment.

### Description of Embodiments

Blood purification apparatuses according to embodiments will be described below with reference to the accompanying drawings. The embodiments to be described below are applied to a drainage process performed after hemodialysis treatment, hemodiafiltration treatment, and the like (hereinafter referred to as dialysis treatment) end. In the drainage process, a liquid remaining inside a blood circuit in the dialysis treatment or the like (residual liquid) is drained. This reduces the residual liquid in the blood circuit and can therefore reduce the cost of disposal of the blood circuit.

In the conventional technique employing a blood circuit formed from a sealed tube, in a case of only driving a liquid delivery pump for delivering a residual liquid in the circuit in a drainage process, the residual liquid in the blood circuit is drained without introducing a medium (such as a liquid and air) into the blood circuit. For this reason, in the conventional technique, the blood circuit may become close to a vacuum state, which may make it impossible to sufficiently push out the residual liquid. The blood purification apparatuses according to the embodiments address such a problem.

### <First Embodiment>

First, a first embodiment will be described. In the first embodiment, an example will be described in which air is introduced into a blood circuit by driving an air pump provided to a blood return side circuit in the blood circuit to drain a residual liquid in the blood circuit. The air introduced into the blood circuit plays a role as a medium for pushing out the residual liquid in the blood circuit.

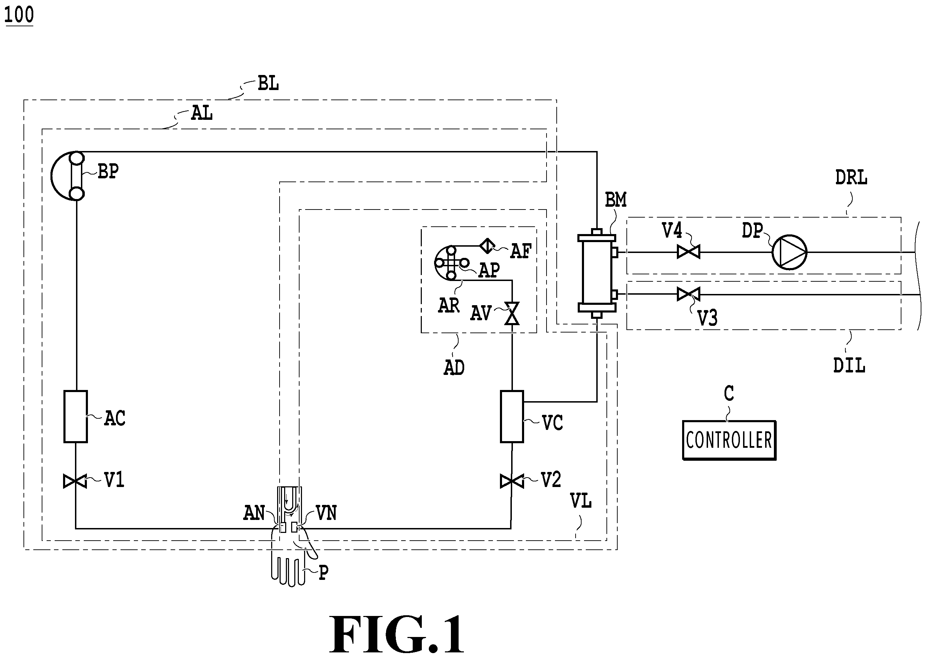

Fig. 1 is a circuit diagram illustrating a configuration of a blood purification apparatus 100 according to the first embodiment. The blood purification apparatus 100 mainly includes a blood circuit (blood line) BL, a blood purifier BM, a dialysate circuit (dialysate line) DIL, a drainage circuit (drainage line) DRL, and a controller C. These constituents are merely examples, and other constituents not illustrated may be included. In Fig. 1, the blood circuit BL, the dialysate circuit DIL, and the drainage circuit DRL are each not strictly defined but correspond to a region surrounded by a dot-dashed line.

The blood circuit BL is a flow route that introduces the blood removed from a patient P into the blood purifier BM and also returns the blood discharged from the blood purifier BM (purified blood) to the body in dialysis treatment. The blood flows from a blood removal side puncture needle AN punctured in a blood removal side (artery) of the patient P to a blood return side puncture needle VN punctured in a blood return side (vein) of the patient P through the blood circuit BL. The blood circuit BL includes a blood removal side circuit (arterial side circuit) AL and a blood return side circuit (venous side circuit) VL. In Fig. 1, the blood removal side circuit AL and the blood return side circuit VL are each not strictly defined but correspond to a region surrounded by a dot-dashed line.

The blood circuit BL forms a sealed flow route (sealed circuit) with a flexible tube through which dialysate and blood can flow. The sealed circuit is a circuit configured (sealed) such that air does not flow from the circuit to / except that air flows from the air introducer to be described below to /. In other words, in the present embodiment, the blood circuit BL is sealed such that air does not flow from the circuit to / in a case where the air introducer is not in operation. The liquid in the blood circuit BL is pulled out by the blood pump to be described below.

The blood removal side circuit AL is a flow route that introduces the blood removed from the patient P into the blood purifier BM. One end of the blood removal side circuit AL is attached to the blood removal side puncture needle AN, and the other end is joined to the blood purifier BM. The blood return side circuit VL is a flow route that returns the blood discharged from the blood purifier BM to the body. One end of the blood return side circuit VL is attached to the blood return side puncture needle VN, and the other end is joined to the blood purifier BM.

The blood removal side circuit AL is provided with an on-off valve (solenoid valve) V1, a blood pump BP, and a blood removal side chamber AC. A flow of the blood in the blood removal side circuit AL is controlled by opening and closing the on-off valve V1.

The blood pump BP delivers the liquid within the blood circuit BL in a direction of movement from the blood removal side circuit AL to the blood return side circuit VL (hereinafter referred to as a normal direction in delivering liquid) or in a direction of movement from the blood return side circuit VL to the blood removal side circuit AL (hereinafter referred to as a reverse direction in delivering liquid). The blood pump BP is formed from a peristaltic pump that includes a stator and a rotor, and the peristaltic pump is driven in such a way as to rotate the rotor. The rotor is rotated by an actuator (not illustrated) such as an electric motor under control of the controller C.

By forward rotation (first rotation) of the blood pump BP, the blood removal side circuit AL pinched between the stator and the rotor is squeezed so as to generate the flow in the normal direction in delivering liquid. Additionally, by reverse rotation (second rotation) of the blood pump BP, the blood removal side circuit AL is squeezed so as to generate the flow in the reverse direction in delivering liquid. The number of rotations of the rotor in the blood pump BP is controlled and detected by closed-loop control using a pulse motor (not illustrated). Note that, instead of the closed-loop control using the pulse motor, the number of rotations of the rotor may be controlled by providing a rotary encoder to the blood pump BP and detecting the rotations of the rotary encoder.

The blood removal side chamber AC is provided to measure the pressure in the blood removal side circuit AL in dialysis treatment, for example. The blood removal side chamber AC also captures air generated as a result of driving the blood pump BP to prevent the air from flowing into the blood purifier BM and causing an air lock. The blood removal side chamber AC includes a blood layer and an air layer in order to capture air. In other words, the blood removal side chamber AC plays a role as a chamber that stores the blood in the blood removal side circuit AL.

The blood return side circuit VL is provided with an on-off valve (a solenoid valve) V2, an air introducer AD, and a blood return side chamber VC. In Fig. 1, the air introducer AD corresponds to a region surrounded by a dot-dashed line. The air introducer AD operates in the drainage process to be described later. A flow of the blood in the blood return side circuit VL is controlled by opening and closing the on-off valve V2.

The air introducer AD includes an air pump AP, an on-off valve (solenoid valve) AV, and an air filter AF. The air pump AP incorporates a rotor and drives the rotor so as to rotate it. The rotor is rotated by an actuator (not illustrated) such as an electric motor under control of the controller C. The number of rotations of the rotor in the air pump AP is controlled and detected by closed-loop control using a pulse motor (not illustrated). Note that, instead of the closed-loop control using the pulse motor, the number of rotations of the rotor may be controlled by providing a rotary encoder to the air pump AP and detecting the rotations of the rotary encoder.

By forward rotation (first rotation) of the air pump AP, air is introduced into the blood return side circuit VL through an air introduction route AR (via the blood return side chamber VC). Additionally, by reverse rotation (second rotation) of the air pump AP, air is drawn from the blood return side circuit VL through the air introduction route AR (via the blood return side chamber VC). The on-off valve AV is provided between the air introduction route AR and the blood return side circuit VL. A flow of air into the blood return side circuit VL is controlled by opening and closing the on-off valve AV. The air filter AF removes dust in the air.

In the present embodiment, the air introducer AD introduces air into the blood return side circuit VL by driving the air pump AP, but is not limited to such a configuration. For example, the air pump AP may not be provided and, for example, the air filter AF may be opened to the atmosphere to set the air filter AF to the atmospheric pressure and thereby introduce air into the blood return side circuit VL.

The blood return side chamber VC is provided to measure the pressure in the blood return side circuit VL in dialysis treatment, for example. The blood return side chamber VC also captures air generated as a result of driving the blood pump BP to prevent the air from flowing into the patient's body through the blood removal side circuit AL. The blood return side chamber VC includes a blood layer and an air layer in order to capture air. In other words, the blood return side chamber VC plays a role as a chamber that stores the blood in the blood return side circuit VL.

Note that, the air introducer AD may play a role in adjusting the liquid level of the liquid in the blood return side chamber VC in dialysis treatment (liquid level adjustment pump), for example. In this case, the air pump AP lowers the liquid level inside the chamber by introducing air into the blood return side chamber VL by forward rotation. Additionally, the air pump AP raises the liquid level inside the chamber by drawing air from the blood return side chamber VL by reverse direction.

The blood purifier BM, which is also referred to as a dialyzer, purifies the blood of the patient P. The blood purifier BM includes a blood purification membrane (not illustrated) that is provided inside. The blood purification membrane is formed from a bundle of hollow fibers having pores on side walls (hollow fiber membrane). An inner side of the blood purification membrane is a blood flow route (not illustrated), and an outer side of the blood purification membrane (hollow fibers) is a dialysate flow route (not illustrated). The blood flowing in the blood purifier BM flows in the blood flow route, and unwanted substances such as uremic substances are removed by passing through the pores in the blood purification membrane by one or both of diffusion and ultrafiltration. The dialysate flowing in the blood purifier BM passes through the dialysate flow route, and the blood is supplemented only with substances such as electrolytes included in the dialysate, which are necessary for a human body, while the substances pass through the pores. Note that, the inner side of the blood purification membrane can also function as the dialysate flow route, and the outer side of the blood purification membrane can also function as the blood flow route.

Note that, a chamber that stores the liquid flowing in the blood purifier BM may be provided in order to measure the pressure at the inlet of the blood purifier BM. This chamber is provided, for example, between the blood purifier BM and the blood pump BP. Additionally, the air from the air introducer AD may be introduced into this chamber.

The dialysate circuit DIL is a flow route from a dialysate supplier (not illustrated) to the blood purifier BM that supplies the blood purifier BM with dialysate from the dialysate supplier. The dialysate circuit DIL forms a sealed flow route (sealed circuit) with a flexible tube through which dialysate can flow. The blood circuit BL and the dialysate circuit DIL are coupled to each other through the blood purification membrane of the blood purifier BM and the blood and the dialysate flow through therethrough toward each other.

The dialysate circuit DIL is provided with an on-off valve (solenoid valve) V3. A flow of the dialysate into the blood purifier BM is controlled by opening and closing the on-off valve V3.

For example, in dialysis treatment, the drainage circuit DRL is a flow route from the blood purifier BM to a drained liquid drainer (not illustrated) that drains the dialysate drained from the blood purifier BM to the drained liquid drainer. The drainage circuit DRL is coupled to the blood circuit BL through the blood purifier BM. The drainage circuit DRL is also used to drain the residual liquid in the blood circuit in the drainage process to be described later. The dialysate circuit DIL forms a sealed flow route (sealed circuit) with a flexible tube through which the drained liquid can flow. The dialysate drainage circuit EL is provided with an on-off valve (solenoid valve) V4 and a drainage pump (water removal pump) DP. A flow of the drained liquid into the drained liquid drainer is controlled by opening and closing the on-off valve V4.

In order to remove excess water from the blood flowing in the blood purifier BM, the drainage pump DP delivers the drained liquid inside the drainage circuit DRL. The drainage pump DP is rotated by an actuator (not illustrated) such as an electric motor under control of the controller C. Note that, the drainage pump DP may not be a water removal pump as long as it is a pump that can implement the present invention. For example, a pressure pump may be employed which pressurizes a pump that supplies and drains the dialysate to and from the blood purifier BM (e.g., a dual pump) from an inlet on the drainage side. In other words, the drainage pump DP means a pump that is driven to draw a liquid from the blood purifier BM.

The controller C is a processing apparatus that instructs and controls the constituents of the blood purification apparatus 100, e.g., controlling the driving of the blood pump BP described above and the like. The controller C includes an arithmetic unit and storage units (storage units such as a RAM and a ROM). A processor such as a CPU or a microcontroller, an ASIC (Application Specific Integrated Circuit), an FPGA (Field Programmable Gate Array), or the like may be mounted as the arithmetic unit, but its form is not limited.

As described above, the operations of the various constituents of the blood purification apparatus 100 are directed and controlled by the controller C, but the various constituents may be automatically instructed and controlled in accordance with a preset program. Also, the various constituents may be instructed and controlled by manual operations by a user. The manual operations are performed via an input apparatus connected to the controller C (operation buttons, a keyboard, a mouse, and the like; not illustrated).

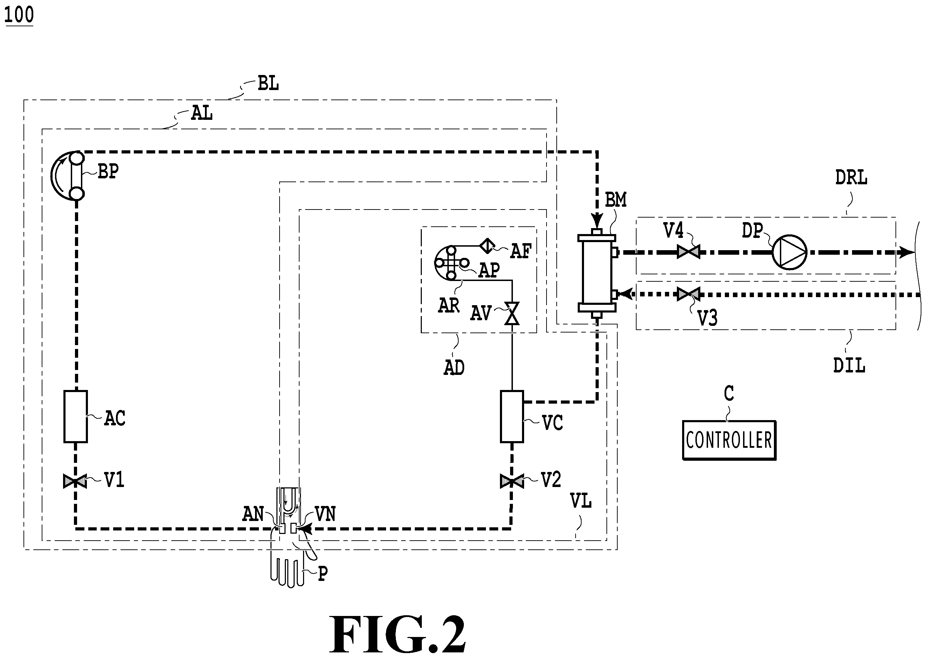

Next, an operation of the blood purification apparatus 100 and flows of the liquids in the blood circuit BL, the dialysate circuit DIL, and the drainage circuit DRL in a case of performing dialysis treatment will be described with reference to Fig. 2. The diagram illustrated in Fig. 2 corresponds to the circuit diagram illustrated in Fig. 1. The blood purification apparatus 100 operates under control of the controller C. In the following diagrams, in a case where the on-off valves are opened, the on-off valves illustrated in the diagrams are indicated with hatching, and in a case where the on-off valves are closed, the on-off valves illustrated in the diagrams are indicated in white.

As illustrated in Fig. 2, in dialysis treatment, the on-off valves V1 to V4 are opened. Additionally, the blood pump BP is driven (rotates forward). By driving the blood pump BP and opening the on-off valves V1 and V2, the blood passes through the blood removal side circuit AL, the blood purifier BM, and the blood return side circuit VL. This flow of the blood is indicated with an arrow of a thick dashed line.

Additionally, a dialysate pump not illustrated is driven. By driving the dialysate pump and opening the on-off valve V3, the dialysate from the dialysate supplier passes through the dialysate circuit DIL and the blood purifier BM. This flow of the dialysate is indicated with an arrow of a thick dotted line.

Additionally, the drainage pump DP is driven. By driving the drainage pump DP and opening the on-off valve V4, excess water and the like from the blood flowing in the blood purifier BM pass through the drainage circuit DRL. This flow of the drained liquid is indicated with an arrow of a thick dot-dashed line.

As described with reference to Fig. 2, in dialysis treatment, the blood purification apparatus 100 operates as described above to take blood out of the patient P, introduce the blood into the blood purifier BM, and introduce the dialysate from the dialysate supplier into the blood purifier BM. The blood is filtered through the blood purifier BM and returned to the patient through the blood return side circuit VL. Note that, the air introducer AD does not operate in the example of the dialysis treatment illustrated in Fig. 2. However, in a case where the air pump AP also plays a role as a liquid level adjustment pump as described above, the air pump AP is driven and the on-off valve AV is opened under control of the controller C.

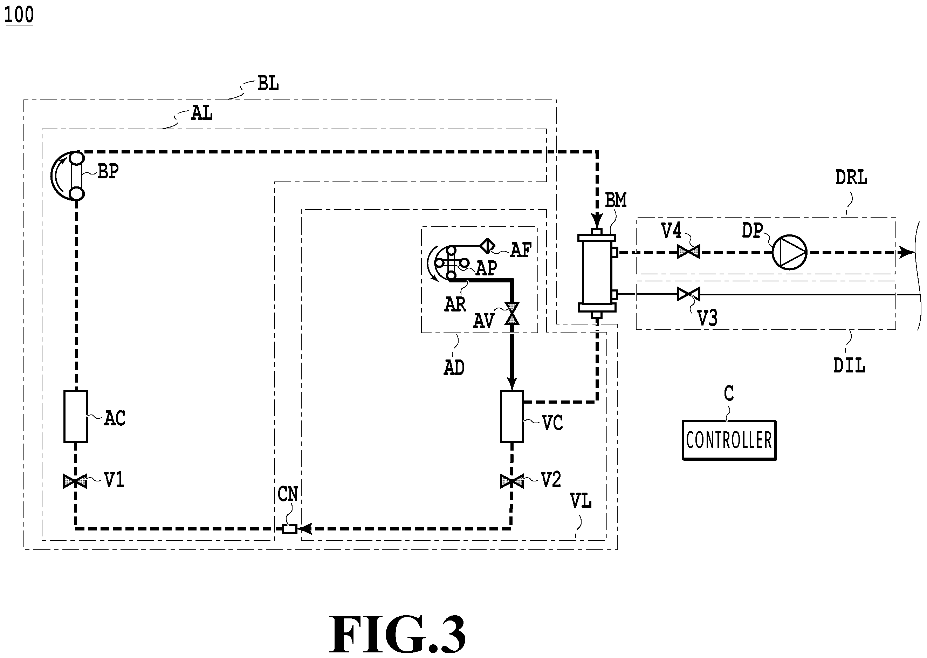

Next, an operation of the blood purification apparatus 100 and a flow of the residual liquid in the blood circuit BL and the drainage circuit DRL in a case of performing a drainage process will be described with reference to Fig. 3. The blood purification apparatus 100 (constituents such as the on-off valves V1 to V5) operates under control of the controller C.

In the drainage process, the blood removal side puncture needle AN and the blood return side puncture needle VN are pulled out of the patient P. Additionally, the blood removal side puncture needle AN is taken out of the blood removal side circuit AL, and the blood return side puncture needle VN is taken out of the blood return side circuit VL. Moreover, the blood removal side circuit AL and the blood return side circuit VL are joined by a connector CN.

As illustrated in Fig. 3, in the drainage process, the on-off valves V1, V2, and V4 are opened and the on-off valve V3 is closed. The blood circuit BL forms a closed loop by opening the on-off valves V1, V2, and V4 and closing the on-off valve V3. The blood pump BP is driven (rotates forward) in such a state. Additionally, the drainage pump DP is driven. Moreover, the on-off valve AV is opened, and the air pump AP rotates forward.

By driving the air pump AP and opening the on-off valve AV, air is introduced into the blood return side circuit VL through the blood return side chamber VC in a direction from the blood return side circuit VL toward the blood removal side circuit AL and the blood purifier BM. This flow of air is indicated with an arrow of a thick solid line. The air introduced from the air introducer AD plays a role as a medium for pushing out the residual liquid in the blood circuit BL in the direction from the blood return side circuit VL toward the blood removal side circuit AL and the blood purifier BM.

Note that, in the present embodiment, the air pump AP is driven to introduce air to the blood return side circuit VL through the blood return side chamber VC, but the present embodiment is not limited to such a configuration. For example, air may be introduced directly into the blood return side circuit VL. In this case, the air introducer AD may be directly joined to the blood return side circuit VL, and the blood return side chamber VC may not be provided in the blood return side circuit VL. Alternatively, as described above, the air pump AP may not be provided to the air introducer AD, and the air filter AF may be opened to the atmosphere to set the air filter AF to the atmospheric pressure and thereby introduce air into the blood return side circuit VL.

By driving the air pump AP and the blood pump BP and opening the on-off valve V1 and V2, the residual liquid in the blood circuit BL passes through the blood return side circuit VL, the blood removal side circuit AL, and the blood purifier BM. In other words, by driving the air pump AP, the residual liquid in the blood circuit BL is pushed out by air as a medium. Additionally, by driving the blood pump BP, the residual liquid is drawn in through the flow route from the blood purifier BM through the blood return side circuit VL and the blood removal side circuit AL to the blood pump BP, and the residual liquid is pushed out through the flow route from the blood pump BP through the blood removal side circuit AL to the blood purifier BM. By driving the drainage pump DP and opening the on-off valve V4, the residual liquid passes through the drainage circuit DRL. In other words, by driving the drainage pump DP, the residual liquid is drawn out of the blood purifier BM. This flow of the blood is indicated with arrows of a thick dashed line.

As described above, the blood circuit BL forms a closed loop, and air is introduced into the blood circuit BL to drain the residual liquid in the blood circuit BL. In particular, the air introducer AD introduces air in the same direction as the direction in which the blood pump BP draws in and pushes out the residual liquid, that is, the introduced air pushes out the residual liquid in the same direction as the direction in which the blood pump BP draws in and pushes out the residual liquid. This enables more efficient drainage of the residual liquid.

Note that, in the present embodiment, an example in which the air introducer AD is provided to the blood return side circuit VL, but the present embodiment is not limited to such a configuration. For example, the air introducer AD may be provided at any position suitable for pushing out the residual liquid in the blood circuit BL in the direction from the blood return side circuit VL toward the blood removal side circuit AL and the blood purifier BM. In other words, the position to provide the air introducer AD is not limited, and air can be introduced into the blood circuit BL (the blood return side circuit VL or the blood removal side circuit AL) by connecting the air introduction route AR to the blood circuit BL.

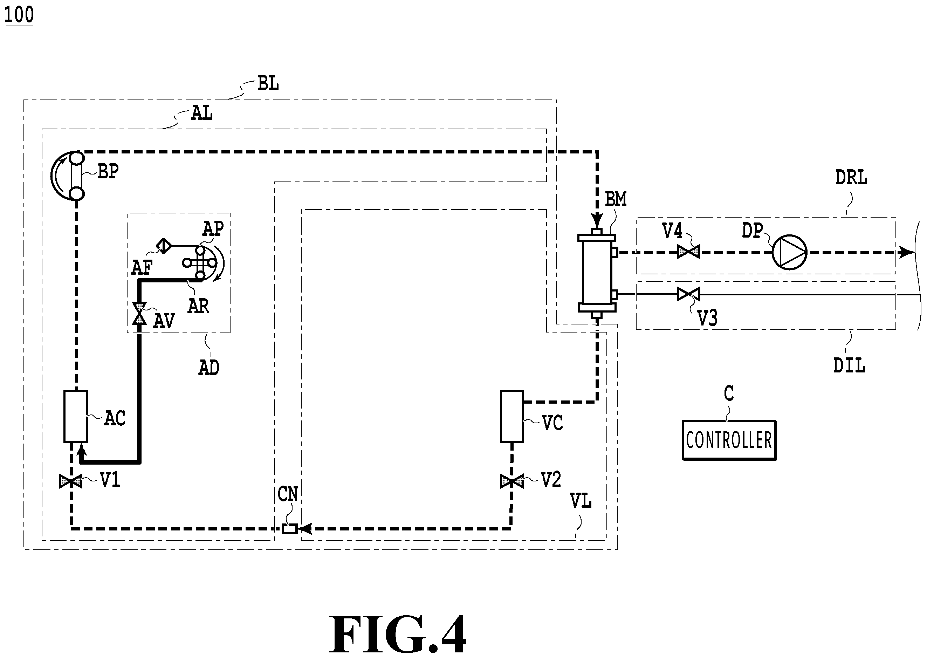

For example, as illustrated in Fig. 4, the air introducer AD may be provided to the blood removal side circuit AL. In this configuration, by driving the air pump AP, air is introduced into the blood removal side circuit AL through the blood removal side chamber AC in a direction toward the blood removal side circuit AL and the blood purifier BM. This flow of air is indicated with an arrow of a thick solid line.

In the configuration in which the air introducer AD is provided to the blood removal side circuit AL, too, the residual liquid in the blood circuit BL is pushed out by air as a medium by driving the air pump AP. Additionally, by driving the blood pump BP, the residual liquid is drawn in through the flow route from the blood purifier BM through the blood return side circuit VL and the blood removal side circuit AL to the blood pump BP, and the residual liquid is pushed out through the flow route from the blood pump BP through the blood removal side circuit AL to the blood purifier BM. By driving the drainage pump DP, the residual liquid passes through the drainage circuit DRL. This flow of the blood is indicated with an arrow of a thick dashed line.

Note that, in the drainage processes illustrated in Figs. 3 and 4, the residual liquid in the blood circuit BL is drawn in and pushed out by driving the blood pump BP, but driving the blood pump BP is not essential. The blood pump BP may not be driven in a case where the residual liquid can be drained only by introducing air with the air introducer AD and driving the drainage pump DP.

Additionally, in the drainage processes illustrated in Figs. 3 and 4, the residual liquid is drawn in from the blood purifier BM by driving the drainage pump DP, but driving the drainage pump DP is not essential. The drainage pump DP may not be driven in a case where the residual liquid can be drained only by driving the blood pump BP. In this case, for example, the drainage circuit DL may not be provided with a pump such as the drainage pump DP and may be provided with only the drained liquid drainer (i.e., an opening).

The blood pump BP and/or the drainage pump DP functions as a liquid delivery pump that delivers and drains the liquid in the blood circuit BP (residual liquid) from the drainage circuit DIL using air introduced from the air introducer AD as a medium for pushing out the liquid in the blood circuit BP (residual liquid).

The blood purification apparatus 100 according to the first embodiment has been described above. According to the first embodiment, the air introducer AD introduces air into the blood circuit BL, so that the air plays a role as a medium for pushing out the residual liquid. Accordingly, it is possible to drain the residual liquid in the blood circuit BL even by driving the liquid delivery pump at a lower output power than that in the conventional technique.

### <Second Embodiment>

Next, a second embodiment will be described. In the first embodiment, an example has been described in which a drainage process is performed by causing the residual liquid in the blood circuit BL to flow in the direction from the blood return side circuit VL toward the blood removal side circuit AL and the blood purifier BM (first direction). In the second embodiment, a drainage process is performed by causing the residual liquid in the blood circuit BL to flow in a direction from the blood return side circuit VL toward the blood purifier BM and the blood removal side circuit AL (second direction), which is the opposite direction to the first direction.

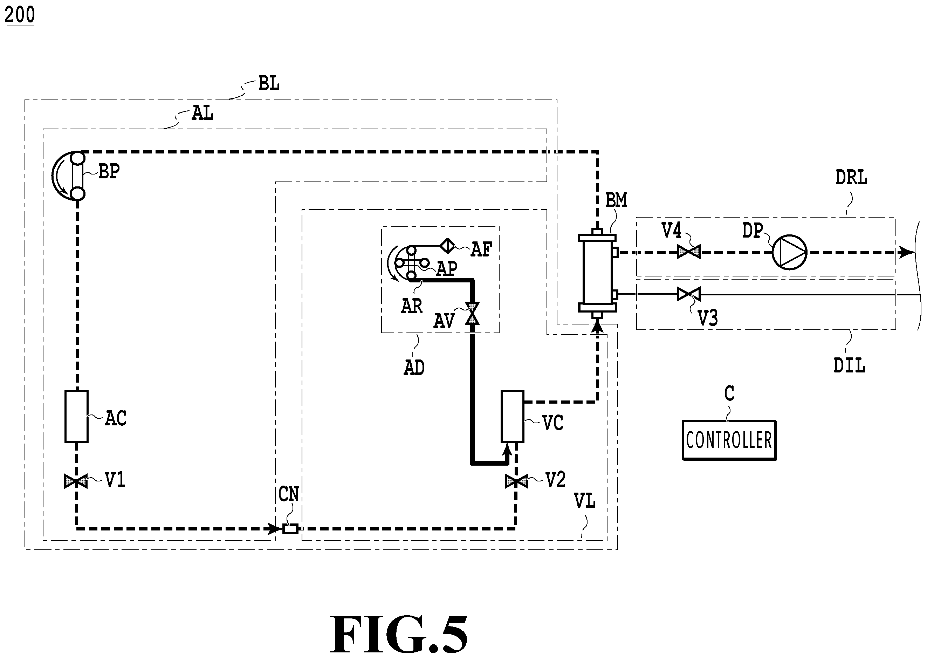

A configuration of a blood purification apparatus 200 according to the second embodiment will be described along with a flow of the residual liquid in the blood circuit in a case where the blood purification apparatus 200 performs the drainage process and the like. An operation of the blood purification apparatus 200 and a flow of the residual liquid in the blood circuit BL and the drainage circuit DRL in a case of performing the drainage process in the second direction will be described with reference to Fig. 5. As illustrated in Fig. 5, the blood purification apparatus 200 differs from the blood purification apparatus 100 in the first embodiment in the position to provide the air introducer AD. Other features are similar to those of the blood purification apparatus 100.

As illustrated in Fig. 5, the air introducer AD is coupled to the blood return side chamber VC. In this configuration, by driving the air pump AP, air is introduced into the blood return side circuit VL through the blood return side chamber VC in the second direction. This flow of air is indicated with an arrow of a thick solid line. The air introduced from the air introducer AD plays a role as a medium for pushing out the residual liquid in the blood circuit BL in the second direction.

In the drainage process in the second direction, the blood pump BP rotates in reverse. Additionally, as described above, the air introducer AD introduces air into the blood return side circuit VL in the second direction. Besides this, the operation of the blood purification apparatus 100 is similar to the operation described with reference to Fig. 3.

As illustrated in Fig. 5, by driving the air pump AP, the residual liquid in the blood circuit BL is pushed out in the second direction by air as a medium. By driving the blood pump BP by reverse rotation, the residual liquid is drawn in through the flow route from the blood purifier BM through the blood removal side circuit AL to the blood pump BP, and the residual liquid is pushed out through the flow route from the blood pump BP through the blood removal side circuit AL and the blood return side circuit VL to the blood purifier BM. By driving the drainage pump DP, the residual liquid passes through the drainage circuit DRL. This flow of the blood is indicated with arrows of a thick dashed line.

As described above, in the drainage process in the second direction too, the air introducer AD introduces air in the same direction as the direction in which the blood pump BP draws in and pushes out the residual liquid, that is, the introduced air pushes out the residual liquid in the same direction as the direction in which the blood pump BP draws in and pushes out the residual liquid. This enables more efficient drainage of the residual liquid.

Note that, in the present embodiment, an example in which the air introducer AD is provided to the blood return side circuit VL has been described, but the air introducer AD may be provided at any position suitable for pushing out the residual liquid in the blood circuit BL in the second direction. In other words, in the second embodiment too, the position to provide the air introducer AD is not limited, and air can be introduced into the blood circuit BL (the blood return side circuit VL or the blood removal side circuit AL) by connecting the air introduction route AR to the blood circuit BL.

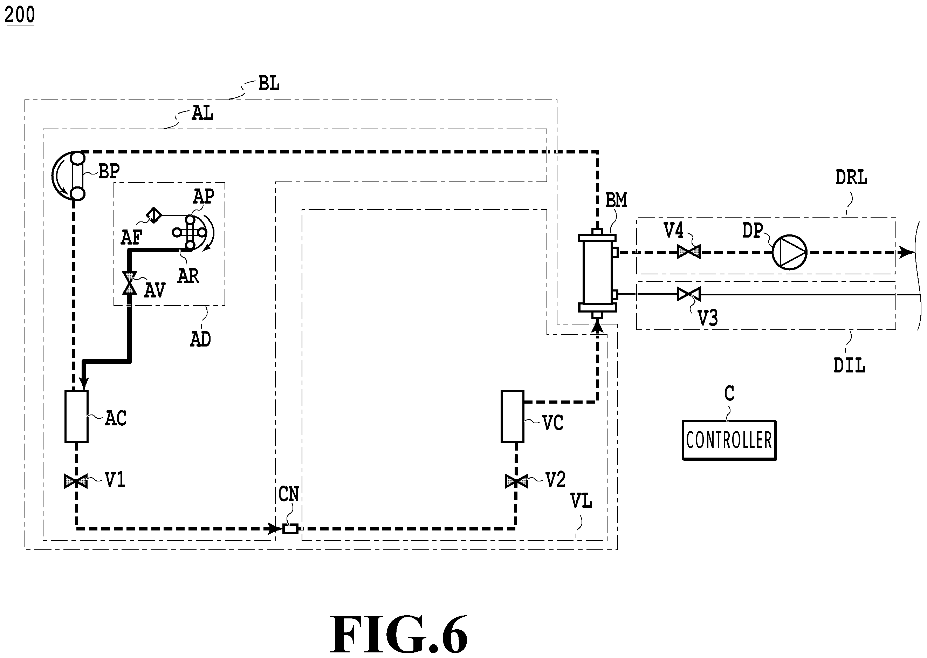

For example, as illustrated in Fig. 6, the air introducer AD may be provided to the blood removal side circuit AL. In this configuration, by driving the air pump AP, air is introduced into the blood removal side circuit AL through the blood removal side chamber AC in the second direction. This flow of air is indicated with an arrow of a thick solid line.

In the configuration in which the air introducer AD is provided to the blood removal side circuit AL, too, the residual liquid in the blood circuit BL is pushed out by air as a medium by driving the air pump AP. Additionally, by driving the blood pump BP, the residual liquid is drawn in through the flow route from the blood purifier BM through the blood removal side circuit AL to the blood pump BP, and the residual liquid is pushed out through the flow route from the blood pump BP through the blood removal side circuit AL and the blood return side circuit VL to the blood purifier BM. By driving the drainage pump DP, the residual liquid passes through the drainage circuit DRL. This flow of the blood is indicated with an arrow of a thick dashed line.

The drainage process in the second direction described above may be performed in two steps along with the drainage process in the first direction described in the first embodiment in any order. Performing the two-step drainage processes in different directions allows sufficient drainage of the residual liquid in the blood circuit BL.

The two-step drainage processes may be performed by a manual operation by the user. In this case, for example, one of the drainage processes may be performed in response to an input into the above-mentioned input apparatus by the user (input of a drainage process start instruction), and the other drainage process may be performed in response to an input into the above-mentioned input apparatus by the user (input of a drainage process switch instruction).

Alternatively, the two-step drainage processes may be performed automatically in accordance with a preset program. For example, one of the drainage processes is performed, and then in a case where a certain time passes, the flow volume of the drained residual liquid reaches a certain volume, or the number of rotations of the air pump AP and/or the blood pump BP to drive it reaches a certain number of rotations, the other drainage process is performed.

The passage of time may be measured by, for example, equipping the controller C with a timer. The flow volume of the residual liquid may be measured by, for example, providing a flowmeter at a position in either the blood circuit BL or the drainage circuit DRL and measuring the flow volume with the flowmeter. Moreover, the number of rotations of the air pump AP and/or the blood pump BP may be measured by, for example, the closed loop control using a pulse motor or the rotary encoder described above.

The blood purification apparatus 200 according to the second embodiment has been described above. According to the second embodiment too, it is possible to drain the residual liquid in the blood circuit BL even by driving the blood pump BP with a lower output power than that in the conventional technique. Also, in the case of performing the two-step drainage processes too, air is introduced into the blood circuit BL in each drainage process. Accordingly, it is possible to continue the drainage process while keeping the blood circuit BL from getting close to a vacuum state.

### <Third Embodiment>

Next, a third embodiment will be described. In the third embodiment, the pressure of air introduced from the air introducer AD is measured, and the introduction of air from the air introducer AD is stopped in a case where the pressure is above a predetermined pressure. Introducing a large amount of air to the blood circuit BL may cause rupture of the tubes forming the circuit or the like. The present embodiment prevents such a phenomenon from occurring.

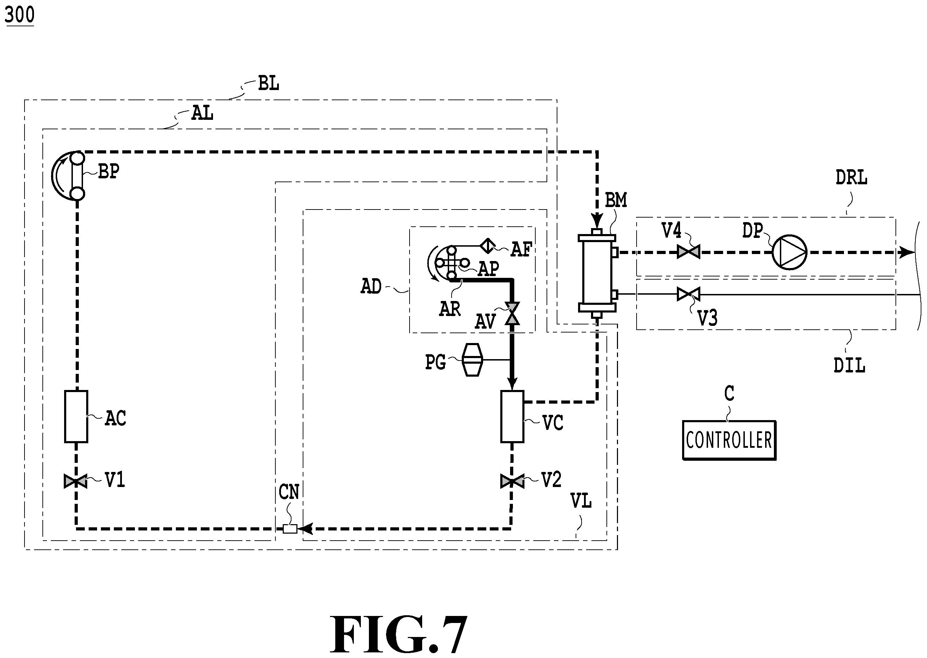

A configuration of a blood purification apparatus 300 according to the third embodiment will be described along with a flow of the residual liquid in the blood circuit in a case where the blood purification apparatus 300 performs a drainage process and the like. The drainage process may be the first drainage process or the second drainage process. An operation of the blood purification apparatus 300 and a flow of the residual liquid in the blood circuit BL and the drainage circuit DRL in a case of performing the drainage process in the first direction will be described with reference to Fig. 7. As illustrated in Fig. 7, the blood purification apparatus 300 differs from the blood purification apparatus 100 in the first embodiment and the blood purification apparatus 200 in the second embodiment in that the blood return side circuit VL is provided with a pressure measurement unit PG. Other features are similar to those of the blood purification apparatus 100 and the blood purification apparatus 200.

The pressure measurement unit PG measures the pressure in the blood circuit BL generated as a result of driving the air pump AP. The measured pressure value is transmitted to the controller C. The controller C determines whether the pressure value is above a preset threshold value and, for example, stops driving the air pump AP in a case where the pressure is above the threshold value. Note that, in the present embodiment, the pressure measurement unit PG is provided to the blood return side circuit VL, but the pressure measurement unit PG is provided to the blood removal side circuit AL in a case where the air introducer AD is provided to the blood removal side circuit AL like the configurations illustrated in Figs. 4 and 6. In this case, the pressure measurement unit PG is provided, for example, between the air introducer AD and the blood removal side chamber AC.

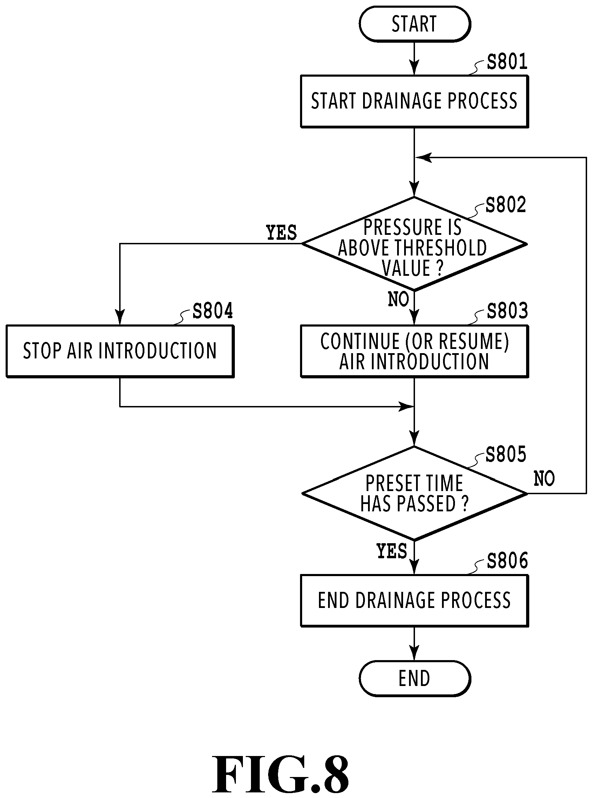

Next, an operation of the blood purification apparatus 300 in the drainage process will be described with reference to the flowchart illustrated in Fig. 8. The drainage process is performed within a preset time and ends in response to elapse of the preset time.

First, step S801, the blood purification apparatus 300 starts the drainage process. The drainage process is started in response to a user input through the input apparatus, for example. To start the drainage process, the controller C sends instructions to the air pump AP, the on-off valves (the on-off valves V1 to V4 and the on-off valve AV), and the blood pump AP. In response to these instructions, the on-off valves V1, V2, V4, and AV open, and the air pump AP and the blood pump BP rotate forward. Additionally, the on-off valve V3 closes. As a result, air from the air introducer AD is introduced to the blood return side circuit VL to perform the drainage process.

In response to starting the drainage process, the controller C determines whether the pressure of the air from the air introducer AD is above the preset threshold value (step S802). The preset threshold value may be a preset fixed value or set by a user input through the input apparatus when the drainage process is performed, and may be 250 mmHg, for example. This determination is performed by the pressure measurement unit PG measuring the pressure in the blood return side circuit and transmitting the measured pressure value to the controller C, and the controller C comparing it with the threshold value. If it is determined that the pressure of the air is not above the preset threshold value (No in step S802), the process moves to step S803. If it is determined that the pressure of the air is above the preset threshold value (Yes in step S802), the process moves to step S804.

In step S803, the controller C sends an instruction to the air pump AP and/or the on-off valve AV so as to introduce the air from the air introducer AD into the blood return side circuit VL. This step resumes the introduction of air from the air introducer AD in a case where the air introduction has been stopped by the process of step S804 to be described later. In response to this instruction, the air pump AP rotates forward and/or the on-off valve AV opens. On the other hand, in a case where the air introduction has not been stopped, the air introduction is continued. In this case, the air pump AP remains driven and the on-off valve AV remains open.

In step S804, the controller C sends an instruction to the air pump AP and/or the on-off valve AV so as to stop the introduction of air from the air introducer AD. In response to this instruction, the air pump AP stops being driven and/or the on-off valve AV closes.

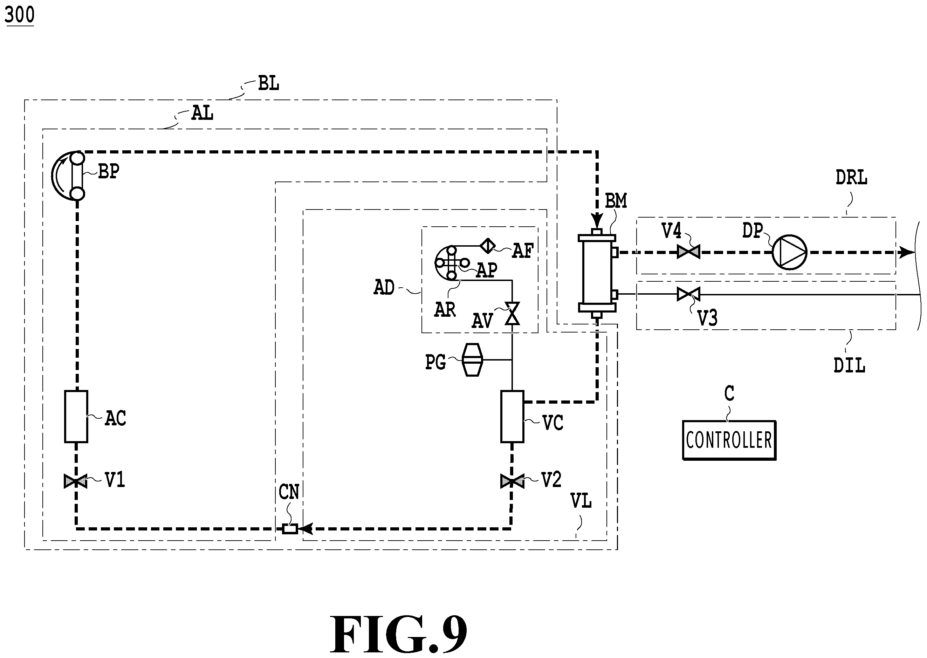

As illustrated in Fig. 9, in the case where the air pump AP stops being driven and the on-off valve AV closes, the introduction of air from the air introducer AD stops. In this state, the residual liquid in the blood circuit BL is drained only by driving the blood pump BP and driving the drainage pump DP. Since a certain amount of air has already been introduced to the blood circuit BL, the residual liquid in the blood circuit BL can be pushed out using the introduced air as a medium without introducing more air. Note that, Fig. 9 illustrates a state where the air pump AP has stopped being driven and the on-off valve AV is closed, but only the air pump AP may stop being driven or only the on-off valve AV may be closed.

As can be understood from Fig. 8, the process in step S802 is repeated. Thus, even in a case where it is determined that the pressure of the air from the air introducer AD is above the threshold value and the air introduction is stopped, the air introduction will be resumed if it is subsequently determined that the pressure of the air from the air AD is not above the threshold value. Likewise, even in a case where it is determined that the pressure of the air from the air introducer AD is not above the threshold value and the air introduction is continued (or resumed), the air introduction will be stopped if it is subsequently determined that the pressure of the air from the air introducer AD is above the threshold value.

Note that, it is not essential to repeat the process in step S802 described above. For example, in a case where it is determined that the pressure of the air from the air introducer AD is above the threshold value and the air introduction is stopped, the process of step S802 may not be performed after that. Then, the drainage process is continued only by driving the blood pump BP and driving the drainage pump DP until a preset time passes.

Next, the controller C determines whether the preset time has passed (step S805). The preset time is a time it takes for the drainage process to end after starting, and may be a preset fixed value or set by a user input through the input apparatus when the drainage process is performed. The passage of time is measured by, for example, equipping the controller C with a timer. If the preset time has not passed (No in step S805), the process moves to step S802. If the preset time has passed (Yes in step S805), the process moves to step S806.

In step S806, the blood purification apparatus 300 ends the drainage process. To end the drainage process, the controller C sends instructions to the air pump AP, the on-off valves (the on-off valves V1, V2, and V4 and the on-off valve AV), and the blood pump AP. In response to these instructions, the on-off valves V1, V2, V4, and AV close, and the air pump AP and the blood pump BP stop being driven.

The present embodiment has been described by applying it to the drainage process described with reference to Fig. 3 (first embodiment), but may be applied to the drainage process described with reference to Fig. 4 and the drainage processes described with reference to Figs. 5 and 6 (second embodiment).

The blood purification apparatus 300 according to the third embodiment has been described above. According to the third embodiment, it is possible to prevent air from being introduced in a large amount into the blood circuit BL in the drainage process. Additionally, even in a case where the air introduction is stopped or continued (or resumed), it is possible to appropriately control the pressure in the blood circuit BL because whether the pressure of the introduced air is above the threshold value continues to be determined.

### <Fourth Embodiment>

Next, a fourth embodiment will be described. In the third embodiment, an example has been described in which the introduction of air from the air introducer AD is stopped in a case where it is determined that the pressure of the air from the air introducer AD is above a threshold value. In the fourth embodiment, the pressure in the blood circuit is lowered in a case where it is determined that the pressure of the air from the air introducer AD is above the threshold value. The configuration of a blood purification apparatus 400 according to the fourth embodiment is similar to that of the blood purification apparatus 300 according to the third embodiment.

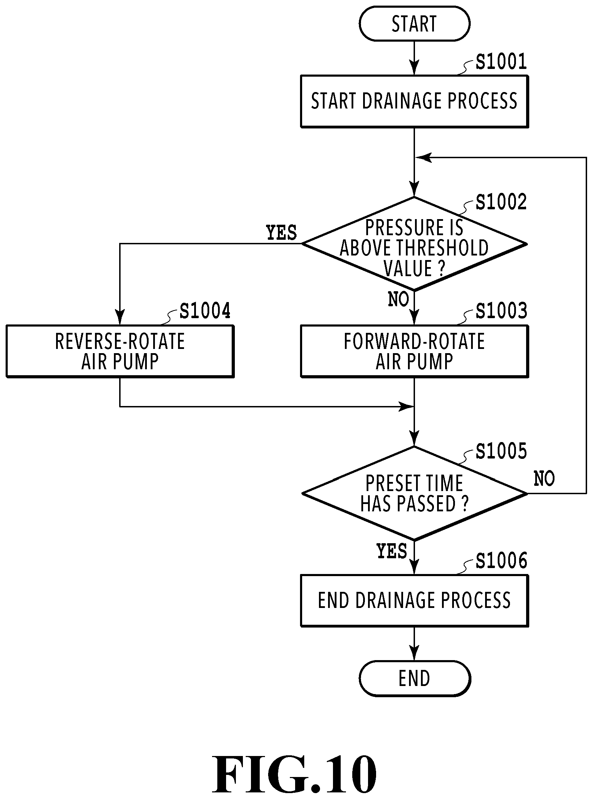

An operation of the blood purification apparatus 400 in a drainage process according to the fourth embodiment will be described with reference to the flowchart illustrated in Fig. 10. The drainage process is performed within a preset time and ends in response to elapse of the preset time. Steps S1001, S1002, S1005, and S1006 illustrated in Fig. 10 are similar to steps S801, S802, S805, and S806 illustrated in Fig. 8, and description thereof is therefore omitted.

In step S 1103, the controller C sends an instruction to the air pump AP so as to introduce the air from the air introducer AD into the blood return side circuit VL. This step causes the air pump AD to rotate forward in order to resume the air introduction in a case where the air pump AD is rotating in reverse in the process of step S1005 to be described later. In response to this instruction, the air pump AP rotates forward. On the other hand, in a case where the air pump AD is not rotating in reverse, the air pump AD continues forward rotation. In this case, the air pump AP keeps rotating forward.

In step S1104, the controller C sends an instruction to the air pump AD so as to lower the pressure in the blood circuit BL. In response to this instruction, the air pump AP rotates in reverse. The reverse rotation of the air pump AP draws air from the blood return side circuit VL, making it possible to lower the pressure in the blood circuit BL.

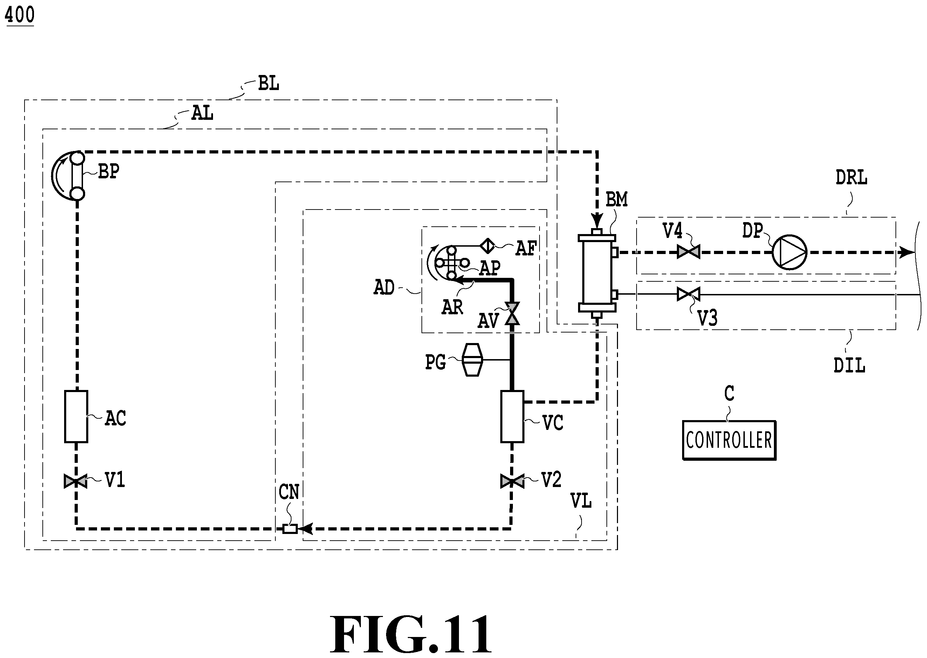

As illustrated in Fig. 11, as the air pump AP rotates in reverse, the air in the blood circuit BL gets drawn into the air introducer. By only driving the blood pump BP and driving the drainage pump DP while drawing in the air in the blood circuit BL in this state, it is possible to continue the drainage process of draining the residual liquid in the blood circuit BL.

Note that, in the present embodiment, the air pump AP is rotated in reverse to lower the pressure in the blood circuit BL. Instead of or in addition to the reverse rotation of the air pump AP, the driving output power of the blood pump BP and/or the drainage pump DP may be increased (e.g., the number of rotations per unit time may be increased), for example. The blood pump BP and the drainage pump DP both play a role in drawing air in along with the residual liquid in the blood circuit BL by being driven (rotated in forward). Hence, increasing the driving output power of either pump can lower the pressure in the blood circuit BL. In other words, in the drainage process, the controller C instructs the blood pump BP and/or the drainage pump DP to draw in the residual liquid in a first draw amount (per unit time) but, in this step, draw in the residual liquid in a second draw amount (per unit time) greater than the first draw amount.

Since the process in step S1102 is repeated, even in a case where, for example, it is determined that the pressure of the air from the air introducer AD is above the threshold value and the air pump AP is rotated in reverse, it may be subsequently determined that the pressure of the air from the air introducer AD is still above the threshold value. In such a case, the controller C may instruct the air introducer AD to increase the driving output power of the air pump AP. In other words, the controller C instructs the air introducer AD to draw in more air from the blood circuit BL than in the previous determination.

In the case of increasing the driving output power of the blood pump BP and/or the drainage pump DP described above, too, the controller C may likewise instruct the blood pump BP and/or the drainage pump DP to increase the driving output power after the second or subsequent determination is made. In other words, the controller C instructs the blood pump BP and/or the drainage pump DP to draw in the residual liquid in a third draw amount (per unit time) greater than the second draw amount. For example, in a case where it is determined that the pressure of the air from the air introducer AD is above the threshold value is a successive number of times, the driving output power may be increased by a value corresponding to that successive number of times.

As above, the blood purification apparatus 400 according to the fourth embodiment has been described. According to the fourth embodiment too, it is possible to prevent air from being introduced in a large amount into the blood circuit BL in the drainage process. Additionally, even in a case where the pressure in the blood circuit BL is lowered, it is possible to appropriately control the pressure in the blood circuit BL because whether the introduced air is above the threshold value continues to be determined.

The above-described embodiments are mere examples. The scopes of the embodiments are not limited only to the described examples. Extra processing and/or constituents may be added to the above-described processes and constituents. Meanwhile, the above-described processes and constituents may be subjected to modifications without departing from the scope of the invention, and particular processes and/or constituents may be omitted. Moreover, the order of the processes described may be changed.

Incidentally, the blood purification apparatuses according to the embodiments are implemented by a computer program to be executed by the controller C. The computer program may be stored in a non-transitory storage medium. Examples of the non-transitory storage medium include a read only memory (ROM), a random access memory (RAM), a register, a cache memory, a semiconductor memory apparatus, a magnetic medium such as a built-in hard disk drive and a removable disk drive, a magneto-optical medium, an optical medium such as a CD-ROM disc and a digital versatile disc (DVD), and so forth.

### Reference Signs List

- 100: blood purification apparatus
- 200: blood purification apparatus
- 300: blood purification apparatus
- 400: blood purification apparatus
- BL: blood circuit
- AL: blood removal side circuit
- AC: blood removal side chamber
- VL: blood return side circuit
- VC: blood return side chamber
- BP: blood pump
- AD: air introducer
- AP: air pump
- AR: air introduction route
- AF: air filter
- AV: on-off valve
- BM: blood purifier
- DIL: dialysate circuit
- DRL: drainage circuit
- DP: drainage pump
- V1: on-off valve
- V2: on-off valve
- V3: on-off valve
- V4: on-off valve
- PG: pressure measurement unit
- AN: blood removal side puncture needle
- VN: blood return side puncture needle
- CN: optical connector
- C: controller

## Claims

1. A blood purification apparatus that drains a residual liquid in a circuit, comprising:
a sealed blood circuit including a blood removal side circuit and a blood return side circuit connected to each other;
a drainage circuit that is connected to the blood circuit and drains a residual liquid in the blood circuit;
an air introducer that is connected to the blood circuit and introduces air into the blood circuit; and
a liquid delivery pump that is provided to at least one of the blood circuit and the drainage circuit and, by rotating, delivers the residual liquid to the drainage circuit from the blood circuit with the introduced air as a medium for pushing out the residual liquid.

2. The blood purification apparatus according to claim 1, wherein the liquid delivery pump is provided to the blood circuit, and draws in the residual liquid and pushes out the residual liquid to the drainage circuit.

3. The blood purification apparatus according to claim 1 or 2, wherein the air introducer includes an air pump that introduces the air into the blood circuit by rotating.

4. The blood purification apparatus according to claim 3, wherein the air introducer is connected to the blood removal side circuit, and the air pump introduces the air in a same direction as a direction in which the liquid delivery pump delivers the residual liquid.

5. The blood purification apparatus according to claim 3, wherein the air introducer is connected to the blood return side circuit, and the air pump introduces the air in a same direction as a direction in which the liquid delivery pump delivers the residual liquid.

6. The blood purification apparatus according to any one of claims 1 to 5, further comprising:
a pressure measurement unit that measures pressure of the air introduced into the blood circuit; and
a controller that determines whether the pressure measured by the pressure measurement unit is above a preset threshold value and causes the air introducer to stop introducing air in a case of determining that the pressure is above the threshold value.

7. The blood purification apparatus according to claim 6, wherein the controller
determines a plurality of times whether the pressure is above the threshold value,
causes the air introducer to stop introducing the air in a case of determining in a first determination that the pressure is above the threshold value, and
causes the air introducer to resume introducing the air in a case of determining in a second determination that the pressure is not above the threshold value.

8. The blood purification apparatus according to any one of claims 3 to 5, wherein:
the air pump introduces the air into the blood circuit by a first rotation, and draws the air from the blood circuit by a second rotation, and
the blood purification apparatus further comprises:
a pressure measurement unit that measures pressure of the air introduced into the blood circuit; and
a controller that determines whether the pressure measured by the pressure measurement unit is above a preset threshold value and causes the air introducer to draw the air from the blood circuit in a case of determining that the pressure is above the threshold value.

9. The blood purification apparatus according to claim 8, wherein the controller
determines a plurality of times whether the pressure is above the threshold value,
causes the air introducer to draw the air from the blood circuit in a case of determining in a first determination that the pressure is above the threshold value, and
in a case of determining in a second determination that the pressure is above the threshold value, causes the air introducer to draw the air from the blood circuit in a larger amount than in the first determination.

10. The blood purification apparatus according to any one of claims 1 to 5 and 8 to 9, wherein
the liquid delivery pump draws in the residual liquid in a first draw amount per unit time, and
the blood purification apparatus further comprises:
a pressure measurement unit that measures pressure of the air introduced into the blood circuit; and
a controller that determines whether the pressure measured by the pressure measurement unit is above a preset threshold value and causes the liquid delivery pump to draw the residual liquid from the blood circuit in a second draw amount per unit time greater than the first draw amount in a case of determining that the pressure is above the threshold value.

11. The blood purification apparatus according to claim 10, wherein the controller
determines a plurality of times whether the pressure is above the threshold value,
causes the air introducer to draw the air from the blood circuit in a case of determining in a first determination that the pressure is above the threshold value, and
in a case of determining in a second determination that the pressure is above the threshold value, causes the liquid delivery pump to draw the residual liquid from the blood circuit in a third draw amount per unit time greater than the second draw amount.

12. The blood purification apparatus according to any one of claims 2 to 11, further comprising a controller that controls the blood purification apparatus so as to execute one of a first drainage process and a second drainage process and then execute the other of the first drainage process and the second drainage process, the first drainage process being a process in which the liquid delivery pump performs a first rotation to draw in and push out the residual liquid in a first direction, and the second drainage process being a process in which the liquid delivery pump performs a second rotation to draw in and push out the residual liquid in a second direction opposite to the first direction.

13. A method of draining a residual liquid in a circuit executed by a blood purification apparatus, the blood purification apparatus including:
a sealed blood circuit including a blood removal side circuit and a blood return side circuit connected to each other; and
a drainage circuit that is connected to the blood circuit and drains a residual liquid in the blood circuit,
the method comprising the steps of:
introducing air into the blood circuit; and
delivering the residual liquid to the drainage circuit from the blood circuit with the introduced air as a medium for pushing out the residual liquid.
